# EUROPÄISCHE PATENTANMELDUNG

(11) **EP 2 127 609 A1**
(43) Veröffentlichungstag der Anmeldung: **02.12.2009**
(21) Anmeldenummer: 09006951.9
(22) Anmeldetag: 25.05.2009
(51) Int. Cl.: A61B 18/20

(54) **Vorrichtung zum Wechsel einer Blitzlampe an einer Blitzlampenaufnahme**

(30) Priorität: 26.05.2008 DE 102008025069
(71) Anmelder: Vondenbusch, Dagmar, 78166 Donaueschingen (DE)
(72) Erfinder: Vondenbusch, Dagmar, 78166 Donaueschingen (DE); Vondenbusch, Alfred, 53474 Bad Neuenahr-Ahrweiler (DE)
(74) Vertreter: Maser, Jochen

(57) **Zusammenfassung**

Die Erfindung betrifft eine Vorrichtung zum Wechseln einer Blitzlampe (11) an einer Blitzlampenaufnahme (12), welche zumindest einen Aufnahmeanschluss (15) aufweist, der von zumindest einem Sockelanschluss (14) der Blitzlampenaufnahme (12) aufgenommen wird, wobei die Blitzlampe (11) und die Blitzlampenaufnahme (12) im montierten Zustand durch eine lösbare Steckverbindung zueinander positioniert sind, wobei eine Handhabungseinrichtung (20) zumindest eine Aufnahmevorrichtung (21) für eine Blitzlampe (11) und zumindest eine weitere Aufnahmevorrichtung (22) für eine Blitzlampenaufnahme (12) umfasst, die relativ zueinander durch eine Schiebebewegung verfahrbar sind und dass die Schiebebewegung von der zumindest einen Aufnahmeeinrichtung (21, 22) zum Lösen und Verbinden der Blitzlampe (11) und der Blitzlampenaufnahme (12) durch eine gemeinsame Antriebseinrichtung (31) ansteuerbar ist, und wobei zumindest eine der Aufnahmevorrichtungen (21, 22) zum kraftfreien Einlegen der Blitzlampe (11) oder Blitzlampenaufnahme (12) ausgebildet ist.

## Beschreibung

Die Erfindung betrifft eine Vorrichtung zum Wechsel einer Blitzlampe an einer Blitzlampenaufnahme, welche in einer Kartusche vorgesehen ist.

Aus der WO 2007/007167 ist eine kartuschenförmige Blitzlampe bekannt geworden, welche in einer Blitzlampenaufnahme austauschbar vorgesehen ist. Diese austauschbare Anordnung einer Blitzlampe zur Blitzlampenaufnahme beruht darauf, dass eine zufriedenstellende Behandlung für eine Haarepilation mit einer solchen Blitzlampe nur dann gewährleistet ist, wenn die Ausgangsleistung der Blitzlampe einen Mindestwert umfasst. Beispielsweise bei einer Behandlung eines Beines sind etwa 500 Lichtblitze erforderlich. Eine solche Behandlung kann ca. in 20 Minuten durchgeführt werden. Unter der Annahme, dass in einem Kosmetikstudio 10 Behandlungen pro Tag durchgeführt werden, wird ein Wechsel der Blitzlampe alle 5 bis 15 Tage erforderlich. In diesem Zeitraum sind ca. 25 000 bis 75 000 Hauptentladungen durch die Blitzlampe erfolgt, und die optische Ausgangsleistung geht dabei so stark zurück, dass die Blitzlampen ausgetauscht werden müssen.

Diese Blitzlampe weist mehrere Aufnahmeanschlüsse auf. Zum einen muss die Blitzlampe mit elektrischer Energie versorgt werden. Zum anderen ist erforderlich, dass die Blitzlampe mit einem Kühlfluid umströmt wird, um eine Überhitzung zu vermeiden. Damit die elektrischen und thermischen Aufnahmeanschlüsse der Blitzlampe gesichert in der Blitzlampenaufnahme angeordnet sind, weisen die Aufnahmeanschlüsse und die korrespondierenden Sockelanschlüsse beim Verbinden und Lösen eine mechanische Reibungskraft auf. Aus diesem Grund sind solche Blitzlampen bzw. Blitzlampenkartuschen nur schwer von der Blitzlampenaufnahme zu lösen oder mit diesen zu verbinden.

Der Erfindung liegt deshalb die Aufgabe zugrunde, eine Vorrichtung zu schaffen, die eine einfache Handhabung, insbesondere einen kräftereduzierten oder kräftefreien Wechsel einer Blitzlampe zur Blitzlampenaufnahme ermöglicht.

Diese Aufgabe wird erfindungsgemäß durch die Merkmale des Anspruchs 1 gelöst. Weitere vorteilhafte Ausgestaltungen und Weiterbildungen sind in weiteren Ansprüchen gegeben.

Durch die erfindungsgemäße Vorrichtung, welche eine Handhabungseinrichtung mit zumindest einer Aufnahmeeinrichtung für die Blitzlampe und zumindest einer Aufnahmeeinrichtung für die Blitzlampenaufnahme aufweist, die relativ zueinander verschiebbar angeordnet sind und mit einer gemeinsamen Antriebsvorrichtung, welche die Schiebebewegung zum Lösen und Verbinden der Blitzlampe und der Blitzlampenaufnahme ansteuert, wobei die zumindest eine Aufnahmevorrichtung zum kraftfreien Einlegen der Blitzlampe oder Blitzlampenaufnahme ausgebildet ist, wird eine einfache und erleichterte Handhabung ermöglicht. Eine Reduzierung des Kraftaufwandes oder sogar eine kräftefreie Montage und Demontage beim Wechsel der Blitzlampe wird möglich, wobei ein kräftefreies Einlegen bzw. Positionieren der Blitzlampe oder Blitzlampenaufnahme in der jeweiligen Aufnahmevorrichtung vorgesehen ist. Folglich wird für solche Blitzlampen zur Pulslichtbehandlung, insbesondere zur Haarepilation, Hautstraffung, Aknebehandlung, Tatooentfernung und Besenreiserentfernung sowohl in Kosmetikstudios als auch im Heimbereich eine einfache Anwendung und ein einfacher Austausch von insbesondere kartuschenförmig vorgesehenen Blitzlampen ermöglicht.

Nach einer bevorzugten Ausgestaltung der Erfindung ist vorgesehen, dass die Aufnahmevorrichtung zumindest eine Auflagefläche aufweist, an welcher zumindest eine Anlagefläche der Blitzlampe oder der Blitzlampenaufnahme zur zumindest formschlüssigen Aufnahme der Blitzlampe und der Blitzlampenaufnahme in der jeweiligen Aufnahmevorrichtung angreift. Dadurch kann nach einem einfachen, insbesondere kraftfreien Einlegen eine sichere Positionierung der Blitzlampe und der Blitzlampenaufnahme in der jeweiligen Aufnahmevorrichtung vorgesehen sein, bevor im Anschluss daran die Antriebseinrichtung betätigt wird, um ein Lösen oder Verbinden der Blitzlampe und Blitzlampenaufnahme durchzuführen. Der formschlüssigen Aufnahme der Blitzlampe und der Blitzlampenaufnahme kann auch eine kraftschlüssige Anordnung überlagert sein.

Die Aufnahmevorrichtung der Handhabungseinrichtung weist gemäß einer bevorzugten Ausführungsform zumindest einen Gehäuseabschnitt zum seitlichen kraftfreien Einschieben der Blitzlampe oder der Blitzlampenaufnahme auf. Beispielsweise kann ein U-förmiger Gehäuseabschnitt vorgesehen sein, um in einfacher Weise die Blitzlampe oder die Blitzlampenaufnahme einzuschieben. Dadurch kann bspw. ein sogenannter Seitenlader geschaffen werden. Alternativ kann vorgesehen sein, dass zumindest ein Gehäuseabschnitt zum kraftfreien Einlegen von oben oder von unten oder von vorne in ein Gehäuseabschnitt vorgesehen ist. Dadurch kann ein sogenannter Toplader oder Frontlader geschaffen werden, der wiederum eine einfache Handhabung ermöglicht. Des Weiteren kann alternativ vorgesehen sein, dass die Blitzlampe und die Blitzlampenaufnahme in einer Aufnahmevorrichtung anordenbar sind, welche bevorzugt zumindest zwei Hälften umfassen, die einen Gehäuseabschnitt bilden, zwischen denen die Blitzlampe oder die Blitzlampenaufnahme angeordnet ist.

Die Handhabungseinrichtung weist gemäß einer weiteren bevorzugten Ausführungsform zwischen den zumindest zwei Aufnahmevorrichtungen eine Führung auf, durch welche die Schiebebewegung entlang einer gemeinsamen Längsachse der Blitzlampe und Blitzlampenaufnahme zum Lösen und Verbinden der Aufnahmeanschlüsse und Sockelanschlüsse geführt wird. Dadurch kann unabhängig der Ansteuerung einer Antriebsbewegung durch die Antriebseinrichtung eine geradlinige Scheibebewegung zum sicheren Verbinden und Lösen der Blitzlampe zur Blitzlampenaufnahme sichergestellt werden.

Die zumindest eine Aufnahmevorrichtung umfasst bevorzugt eine Verriegelungseinrichtung zum Fixieren der Blitzlampe und der Blitzlampenaufnahme. Dadurch kann während der Montage und Demontage sichergestellt werden, dass die Blitzlampe und die Blitzlampenaufnahme gesichert während der Schiebebewegung von der jeweiligen Aufnahmevorrichtung aufgenommen werden, so dass eine Beschädigung der Aufnahmeanschlüsse bzw. Sockelanschlüsse verhindert ist.

Nach einer weiteren bevorzugten Ausgestaltung der Erfindung ist vorgesehen, dass die Antriebseinrichtung einen Hebelmechanismus zur Kraftreduzierung der Schiebebewegung beim Lösen und Verriegeln der lösbaren Steckverbindung zwischen der Blitzlampe und der Blitzlampenaufnahme umfasst. In Abhängigkeit der Größe bzw. Länge und der Anzahl der Hebel kann die Kraftreduzierung eingestellt werden. Dabei ist bevorzugt vorgesehen, dass zu Beginn der Lösephase bzw. am Ende der Verriegelungsphase ein geringer Verschiebeweg bei einem großen Hebelweg erfolgt, um mit möglichst geringer Kraft eine Montage und Demontage der Blitzlampe von oder zur Blitzlampenaufnahme zu ermöglichen.

Bevorzugt ist vorgesehen, dass der Hebelmechanismus zumindest einen Schwenkhebel umfasst, dessen Schwenkachse rechtwinklig zur Schiebebewegung ausgerichtet ist. Diese Schwenkachse kann sowohl an der Aufnahmevorrichtung zur Aufnahme der Blitzlampe als auch der Aufnahmevorrichtung zur Aufnahme der Blitzlampenaufnahme vorgesehen sein. Die Geometrie des Schwenkhebels wird auf die Geometrie der weiteren Steuerelemente angepasst. Beispielsweise kann ein L-förmiger Schwenkhebel vorgesehen sein, wobei der lange Schenkel des L-förmigen Schwenkhebels zur manuellen Krafteinleitung verwendet wird und der kurze Schwenkhebel das Lösen und Verbinden der Blitzlampe zur Blitzlampenaufnahme bzw. die Schiebebewegung zwischen den Aufnahmevorrichtungen bewirkt. Alternativ kann auch ein Schwenkhebel vorgesehen sein, dessen Stift zur Ansteuerung einer Schwenkbewegung zwischen der Schwenkachse und einem Angriffspunkt zum Schwenken des Schwenkhebels liegt. Des Weiteren kann alternativ vorgesehen sein, dass der Schwenkhebel als Teil eines Kniehebels zur Ansteuerung der Schiebebewegung ausgebildet ist. Des Weiteren kann der Schwenkhebel einen Öffnungsschlitz aufweisen, in welchen ein Stift verschiebbar eingreift, der an der anderen Aufnahmevorrichtung vorgesehen ist.

Nach einer weiteren alternativen Ausgestaltung des Hebelmechanismus ist vorgesehen, dass dieser durch eine Kreisscheibe mit einem in eine Kulisse eingreifenden Führungsstift ausgebildet ist. Dadurch ist eine weitere einfache mechanische Anordnung für einen kraftreduzierenden Antrieb vorgesehen.

Eine weitere alternative Ausgestaltung der Erfindung sieht vor, dass die Antriebseinrichtung als Spindelantrieb oder Zahnstangenantrieb ausgebildet ist. Diese Anordnung ermöglicht eine raumsparende Anordnung. Durch ein an der Spindel fest angeordnetes oder abnehmbares Werkzeug kann durch eine einfache Drehbewegung ein Lösen und Verbinden der Blitzlampe und Blitzlampenaufnahme erfolgen. In Abhängigkeit der Größe des Werkzeuges bzw. der Länge des Drehhebels kann wiederum eine Kraftreduzierung erfolgen.

Die Antriebseinrichtung ist gemäß einer alternativen Ausgestaltung als Antriebsmotor ausgebildet, der über einen Spindelantrieb, einen Zahnstangenantrieb, einen Hebelmechanismus oder ein Kurvengetriebe die Schiebebewegung zwischen den zumindest zwei Aufnahmevorrichtungen antreibt. Durch einen solchen motorischen Antrieb ist für den Benutzer ein kräftefreies Austauschen der Blitzlampe ermöglicht. Durch die bevorzugte kraftfreie Anordnung der Blitzlampe und der Blitzlampenaufnahme in der jeweiligen Aufnahmevorrichtung wird im besonderen Maße eine einfache Handhabung ermöglicht.

Der Antriebsmotor ist bevorzugt elektrisch, hydraulisch, pneumatisch oder elektromagnetisch ansteuerbar. Die Antriebsart kann in Abhängigkeit der weiteren Peripherie ausgewählt werden.

Die Antriebseinrichtung ist gemäß einer weiteren bevorzugten Ausführungsform in einer die Blitzlampenaufnahme aufnehmenden Aufnahmevorrichtung vorgesehen. Gemäß einer alternativen Ausführungsform kann diese Antriebseinrichtung auch an der zumindest einen die Blitzlampe aufnehmenden Aufnahmevorrichtung vorgesehen sein.

Nach einer weiteren bevorzugten Ausgestaltung der Erfindung ist vorgesehen, dass die Handhabungseinrichtung zumindest einen Sensor umfasst, der nach dem vollständigen Verbinden der Blitzlampe und der Blitzlampenaufnahme den Kühlkreislauf und die Spannungsversorgung für die Blitzlampe freigibt. Bevorzugt wird nach dem Schließen und Verriegeln des Gehäuses die Versorgung der Blitzlampe mit Kühlfluid und elektrischem Strom freigegeben. Dadurch kann die Gefahr einer nur teilweisen Montage und einer sich daraus möglicherweise ergebenden Gefahr verhindert werden. Zum Betreiben der Blitzlampe wird unter Druck ein Kühlfluid der Kühllampe zugeführt und gleichzeitig wird die Blitzlampe mit einer hohen Spannung von bis zu 800 V betrieben. Deshalb ist ein unbeabsichtigtes Lösen zu vermeiden.

Nach einer weiteren bevorzugten Ausgestaltung der Erfindung ist vorgesehen, dass die Handhabungseinrichtung zumindest einen Sensor aufweist, der ein Öffnen des Gehäuses oder eine Schiebebewegung der zumindest einen Aufnahmevorrichtung frei gibt, sobald die Blitzlampe spannungsfrei und die Kühlwasserschläuche, die zur Blitzlampe führen, drucklos geschaltet sind. Durch diesen Sensor wird eine Schutzmaßnahme getroffen, um ein unbeabsichtigtes Öffnen des Gehäuses und Entfernen der Blitzlampe aus der Blitzlampenaufnahme zu vermeiden.

Beispielsweise kann der Sensor als Detektor des Simmerstromes der Blitzlampe oder als Spannungsdetektor ausgeführt werden und somit in einfacher Weise erkennen, ob die elektrischen Anschlüsse spannungsfrei geschaltet sind. Weitere Prinzipien zur Erkennung der spannungsfreien Anordnung der Blitzlampe und drucklosen Kühlwasserschläuche können ebenfalls vorgesehen sein.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1a: eine schematische Darstellung zur prinzipiellen Ausgestaltung einer Aufnahmevorrichtung für eine Blitzlampe und einer Blitzlampenaufnahme einer erfindungsgemäßen Vorrichtung,
- Figur 1b: eine schematische Darstellung der Blitzlampe von unten,
- Figuren 2a und b: eine schematische Seitenansicht und Draufsicht auf eine erste Ausführungsform der erfindungsgemäßen Vorrichtung,
- Figuren 3a und b: eine schematische Seitenansicht und Draufsicht einer alternativen Ausführungsform zu Figur 2,
- Figuren 4a und b: eine schematische Seitenansicht und Draufsicht einer weiteren alternativen Ausführungsform zu Figur 2,
- Figuren 5a und b: eine schematische Seitenansicht und Draufsicht einer weiteren alternativen Ausführungsform zu Figur 2,
- Figur 5c: eine schematische Seitenansicht einer weiteren alternativen Ausführungsform zu den Figuren 5a und b,
- Figur 6: eine schematische Seitenansicht einer weiteren alternativen Ausführungsform zu Figur 2,
- Figur 7: eine schematische Ansicht von oben auf eine weitere alternative Ausführungsform der Vorrichtung mit einem Antriebsmotor,
- Figur 8: eine schematische Seitenansicht der Ausführungsform zu Figur 7 mit einer externen Energieversorgung und Ansteuereinheit,
- Figuren 9a und b: eine weitere schematische Seitenansicht und Schnittansicht einer alternativen Ausführungsform zu Figur 7,
- Figuren 10a, b und c: weitere schematische Ansichten einer weiteren alternativen Ausführungsform der Erfindung mit einer extern angebrachten Antriebseinrichtung,
- Figuren 11a, b und c: weitere schematische Ansicht einer weiteren alternativen Ausführungsform mit einer extern angebrachten Antriebseinrichtung,
- Figuren 12a und b: schematische Seitenansichten einer weiteren alternativen Ausführungsform mit einer extern angebrachten Antriebseinrichtung,
- Figuren 13a und b: schematische Seitenansichten einer weiteren alternativen Ausführungsform der Vorrichtung,
- Figuren 14a, b und c: weitere schematische Schnittdarstellungen einer alternativen Ausführungsform der erfindungsgemäßen Vorrichtung und
- Figur 15: eine schematische Schnittdarstellung der Ausführungsform der Vorrichtung zu Figur 13 mit Darstellung von Sensor- und Aktoreinbauorten eines Sicherungssystems.

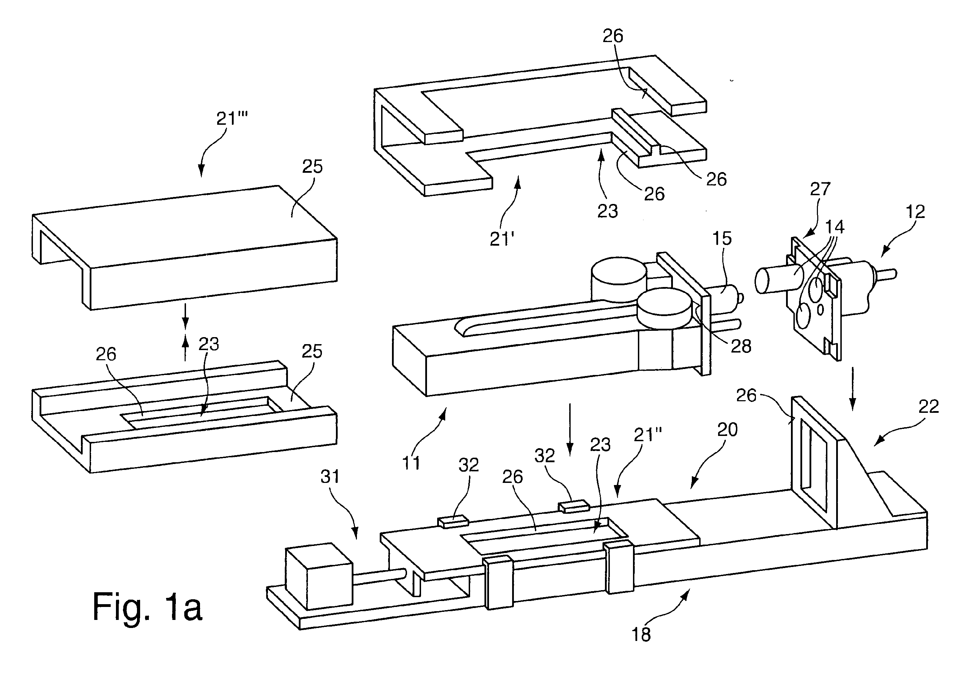

In Figur 1a ist perspektivisch eine Blitzlampe 11 dargestellt, welche getrennt von einer Blitzlampenaufnahme 12 angeordnet ist. Die Blitzlampenaufnahme 12 weist Sockelanschlüsse 14 für die Zuführung der Kühlflüssigkeit, für die Spannungsversorgung der Lampe, für die Hochspannungselektroden sowie für eine elektrische Kontaktierung eines Chips auf, welche in Verbindung mit Aufnahmenanschlüssen 15 der Blitzlampe 11 verbindbar sind. Diese Blitzlampe 11 und die dazugehörige Blitzlampenaufnahme 12 ist aus der WO 2007/007167 bekannt.

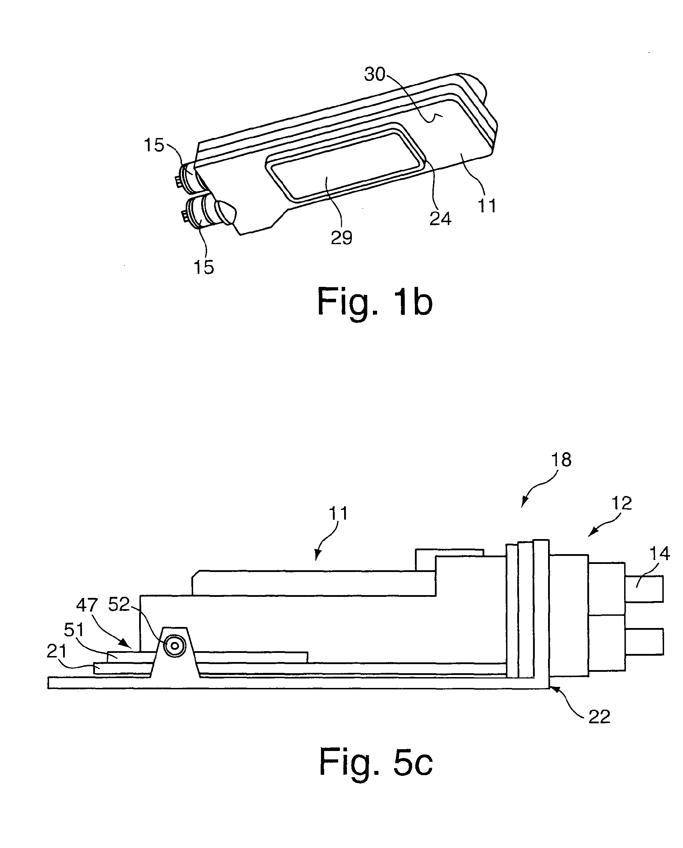

Zum Verbinden der Blitzlampe 11 mit der Blitzlampenaufnahme 12 oder zum Lösen der Blitzlampe 11 von der Blitzlampenaufnahme 12 zum Austausch der Blitzlampe 11, ist eine erfindungsgemäße Vorrichtung 18 vorgesehen. Diese erfindungsgemäße Vorrichtung 18 ist in Figur 1 nur prinzipiell dargestellt. Die Vorrichtung 18 umfasst zumindest eine Aufnahmevorrichtung 21 für eine Blitzlampe 11 und eine Aufnahmevorrichtung 22 für eine Blitzlampenaufnähme 12, wobei beispielhaft drei prinzipielle Ausführungsformen 21', 21" und 21"' in Figur 1 dargestellt sind.

Die Aufnahmevorrichtung 21' ist bspw. als Seitenlader dargestellt, in dem diese Aufnahmevorrichtung 21' einen U-förmigen Gehäuseabschnitt aufweist, so dass durch seitliches Einschieben der Blitzlampe 11 eine Aufnahme der Blitzlampe 11 in die Aufnahmevorrichtung 21 ermöglicht ist. In einer U-förmigen Ausnehmung 23 ist ein umlaufender Bund 24 eines optischen Fensters 29 für die Blitzlampenröhre der Blitzlampe 11 anordenbar. Eine solche Ansicht von unten auf die Blitzlampe 11 ist in Figur 1b dargestellt. Durch das optische Fenster 29 der Blitzlampe 11 erfolgt ein Lichtaustritt senkrecht zur Verschiebebewegung der Aufnahmevorrichtung 21 oder 22.

In Figur 1a ist des Weiteren eine prinzipielle Anordnung der Aufnahmevorrichtung 21" dargestellt, welche als Toplader ausgebildet ist. Diese Anordnung ermöglicht ein einfaches Einlegen der Blitzlampe 11 von oben in die Aufnahmevorrichtung 21". Dabei greift der umlaufende Bund 24 der Blitzlampe 11 in die fensterförmige Ausnehmung 23. Des Weiteren zeigt die Figur 1 eine prinzipielle Anordnung der Aufnahmevorrichtung 21''', welche aus zwei Hälften 25 besteht, in die die Blitzlampe 11 eingelegt werden kann. Eine der beiden Hälften 25 weist bevorzugt Stifte auf, um die zweite Hälfte 25 aufzunehmen und die Position zur ersten Hälfte 25 zu halten.

Diese prinzipiellen Anordnungsmöglichkeiten und Aufnahmemöglichkeit der Aufnahmevorrichtung 21 für die Blitzlampe 11 gelten in Analogie auch für die Aufnahmevorrichtung 22 zur Aufnahme der Blitzlampenaufnahme 12, welche beispielhaft und schematisch als Toplader dargestellt ist. Zumeist ist vorgesehen, dass die Blitzlampenaufnahme 12 fest mit der Aufnahmevorrichtung 22 verbunden ist.

Diese Aufnahmevorrichtungen 21, 22 haben bevorzugt gemeinsam, dass ein kraftfreies Einlegen der Blitzlampe 11 und der Blitzlampenaufnahme 12 in die Aufnahme 23 ermöglicht ist. In den Aufnahmevorrichtungen 21, 22 sind die Auflageflächen 26 vorgesehen, an welchen zumindest abschnittsweise eine Anlagefläche 28 der Blitzlampe 11 und/oder eine Anlagefläche 27 der Blitzlampenaufnahme 12 formschlüssig und/oder kraftschlüssig angreift.

Die erfindungsgemäße Vorrichtung 18 umfasst zumindest die Aufnahmevorrichtung 21 für die Blitzlampe 11 und zumindest die Aufnahmevorrichtung 22 für die Blitzlampenaufnahme 12, die die Handhabungseinrichtung 20 bilden und relativ zueinander durch eine Schiebebewegung verfahrbar sind. Hierfür ist eine Antriebseinrichtung 31 vorgesehen, welche in den nachfolgenden Figuren näher erläutert ist und ermöglicht, dass zumindest in der Anfangsphase beim Lösen der lösbaren Steckverbindung zwischen der Blitzlampe 11 und der Blitzlampenaufnahme 12 und in der Endphase beim Verbinden der lösbaren Steckverbindung zwischen der Blitzlampe 11 und der Blitzlampenaufnahme 12 eine zumindest kraftreduzierte zwangsweise Schiebebewegung erfolgt. Zwischen den Aufnahmevorrichtungen 21 und 22 ist zumindest eine Führung 32, insbesondere Schiebeführung, vorgesehen, so dass während der Schiebebewegung die Blitzlampe 11 und die Blitzlampenaufnahme 12 bezüglich deren Längsachsen entlang einer gemeinsamen Längsachse verschiebbar zueinander geführt sind.

Die in der Figur 1a nur prinzipiell und schematisch dargestellten Aufnahmevorrichtungen 21, 22 der erfindungsgemäßen Vorrichtung 11 und deren weiteren Komponenten wird nachfolgend anhand der weiteren Figuren beispielhaft näher beschrieben.

Für eine gute optische Ankopplung eines optischen Fensters 29 der Blitzlampe 11 an die weiteren optischen Komponenten zur Lichtführung ist vorzugsweise vorgesehen, dass das optische Fenster 29 an den optischen Komponenten, beispielsweise der Haarentfernungsvorrichtung, anliegen, d. h. es soll kein Luftspalt vorhanden sein. In diesem Fall ist ein Verschieben der Blitzlampe 11 nicht vorgesehen, da das optische Fenster 29 dadurch beschädigt werden könnte. Die Aufgabe des Blitzlampenwechsels wird dann dadurch gelöst, dass die Blitzlampe 11 senkrecht zum Verschiebeweg in die Ausnehmung 23 eingelegt wird und der Sockelanschluss 14 über die Aufnahmevorrichtung 22 zur Blitzlampe 11 hinbewegt wird bzw. von dieser entfernt wird. Durch das senkrechte Einlegen von oben der Blitzlampe 11 ist ermöglicht, dass das optische Fenster 29 kratzfrei auf der weiteren optischen Komponente aufliegen kann. Alternativ kann vorgesehen sein, dass zwischen dem optischen Fenster 29 der Blitzlampe 11 und den optischen Komponenten ein geringer Luftspalt vorgesehen ist, so dass dann die Blitzlampe 11 durch eine Verschiebebewegung vom Sockelanschluss 14 getrennt werden kann.

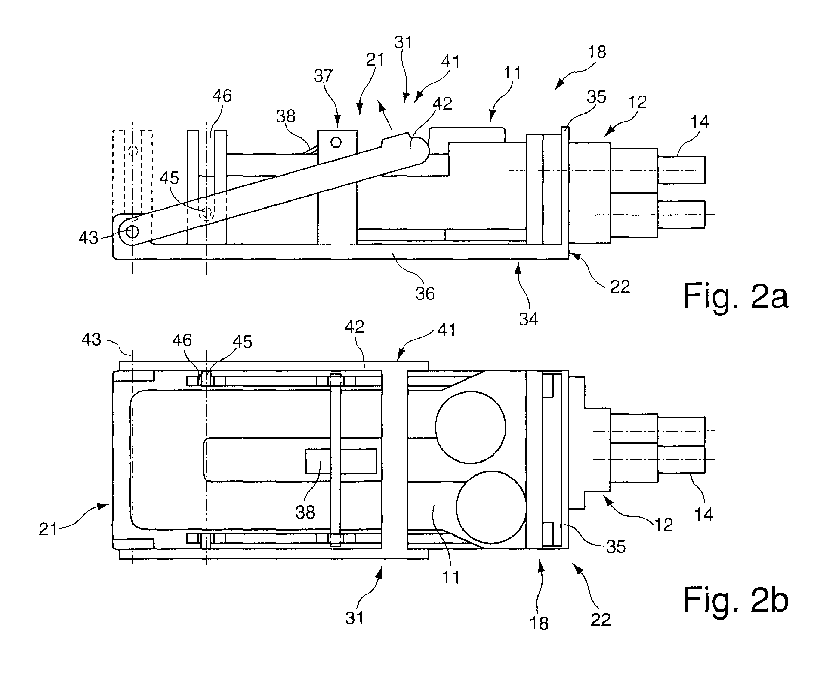

In Figur 2a und b ist eine schematische Seitenansicht und Draufsicht einer ersten erfindungsgemäßen Ausführungsform der Vorrichtung 18 dargestellt. Die Aufnahmevorrichtung 22 zur Aufnahme der Blitzlampenaufnahme 12 weist bspw. ein L-förmiges Gehäuse 34 auf, wobei ein kurzer Schenkel 35 eine U-förmige Ausnehmung aufweist, in welche die Blitzlampenaufnahme 12 einsetzbar ist. Auf einem langen Schenkel 36 des L-förmigen Gehäuses 34, der auch als Grundplatte ausgebildet ist, liegt die zweite Aufnahmevorrichtung 21 auf. Diese Aufnahmevorrichtung 21 nimmt die Blitzlampe 11 auf. Durch eine Verriegelungseinrichtung 37, welche bspw. als lösbarer Stift mit einem Niederhalteelement 38 ausgebildet ist, wird in die Blitzlampe 11 zur Aufnahmevorrichtung 21 positioniert. Die Blitzlampe 11 kann kraftfrei in die Aufnahmevorrichtung 21 von oben eingelegt werden. Die Antriebseinrichtung 31 zum Lösen einer lösbaren Steckverbindung zwischen der Blitzlampe 11 und der Blitzlampenaufnahme 12 umfasst bspw. einen Hebelmechanismus 41. Ein Hebel bzw. Hebelarm 42 ist um eine Schwenkachse 43 an der Aufnahmevorrichtung 22 schwenkbar aufgenommen. Zwischen der Schwenkachse 43 und einem gegenüberliegenden Ende des Hebelarmes 42 ist ein Führungsstift 45 vorgesehen, der in einer Kulisse 46 während einer Schwenkbewegung des Hebelarmes 42 auf und ab geführt wird. Durch den Abstand des Führungsstiftes 45 zur Schwenkachse 43 und der Länge des Hebelarmes 42 kann die Kraftreduzierung des Hebelmechanismus 41 eingestellt und ausgelegt werden. Der Kraftverlauf beim Schwenken des Hebelarmes 42 ist besonders günstig, da der anfänglich große Kraftaufwand zum Lösen der Blitzlampe 11 mit einem langen Hebelweg einhergeht und deshalb stark reduziert wird. Auch der hohe Kraftaufwand kurz vor Erreichen der Endstellung beim Einschieben der Blitzlampe 11 in die Blitzlampenaufnahme 12 wird durch die nichtlineare Hebelwirkung stark reduziert.

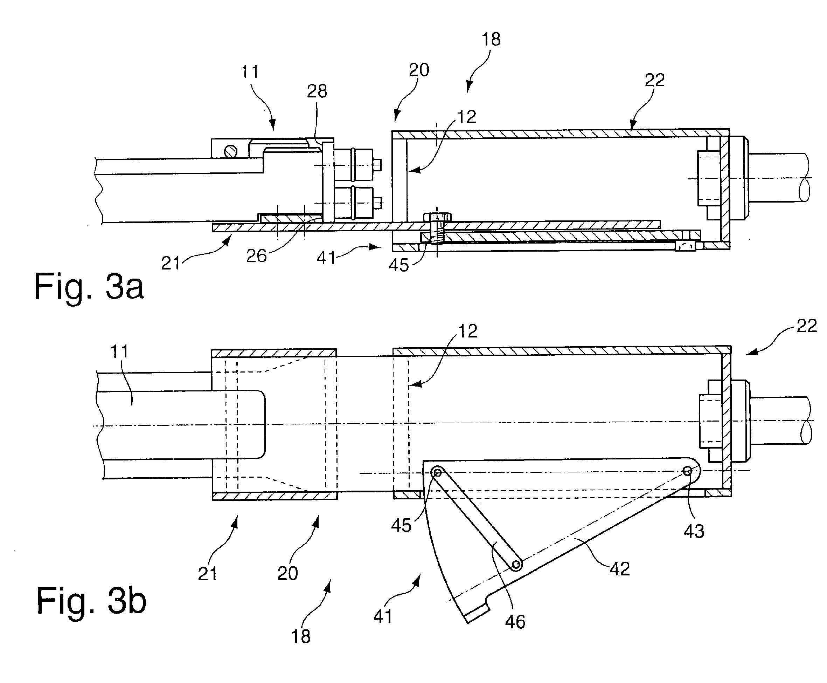

In Figur 3a ist eine schematische Seitenansicht einer alternativen Ausführungsform der Vorrichtung 18 dargestellt. Figur 3b zeigt eine Draufsicht auf die Vorrichtung 18 gemäß Figur 3a. Die Blitzlampe 11 ist form- und/oder kraftschlüssig von der Aufnahmeeinrichtung 21 aufgenommen. Die Blitzlampenaufnahme 12 ist in der Aufnahmevorrichtung 22 form- und/oder kraftschlüssig vorgesehen. Zwischen der Aufnahmevorrichtung 21 und der Aufnahmevorrichtung 22 ist wiederum ein Hebelmechanismus 41 vorgesehen, der an der Aufnahmevorrichtung 22 eine Schwenkachse 43 umfasst. Diese Schwenkachse 43 ist wiederum senkrecht zur Verschiebebewegung ausgerichtet, wobei diese um 90° gedreht gegenüber der Schwenkachse 43 im Ausführungsbeispiel gemäß den Figuren 2a und 2b ausgerichtet ist. Der Hebelarm 42 weist bspw. eine Kulisse 46 auf, in der ein Führungsstift 45 geführt ist, welcher an der Aufnahmevorrichtung 21 fixiert ist. Durch diese Schwenkbewegung des Hebelarmes 42 erfolgt eine Schiebebewegung entlang des Verschiebeweges, der durch die Kulisse 46 bestimmt ist. Die Kulisse 46 kann dabei wiederum auf die Löse- oder Verbindungsphasen zwischen der Blitzlampe 11 und der Blitzlampenaufnahme 12 durch eine Anpassung des Krümmungsverlaufs eingestellt werden. Als bevorzugte Aufnahmevorrichtungen 21, 22 kommen Toplader und Seitenlader zum Einsatz.

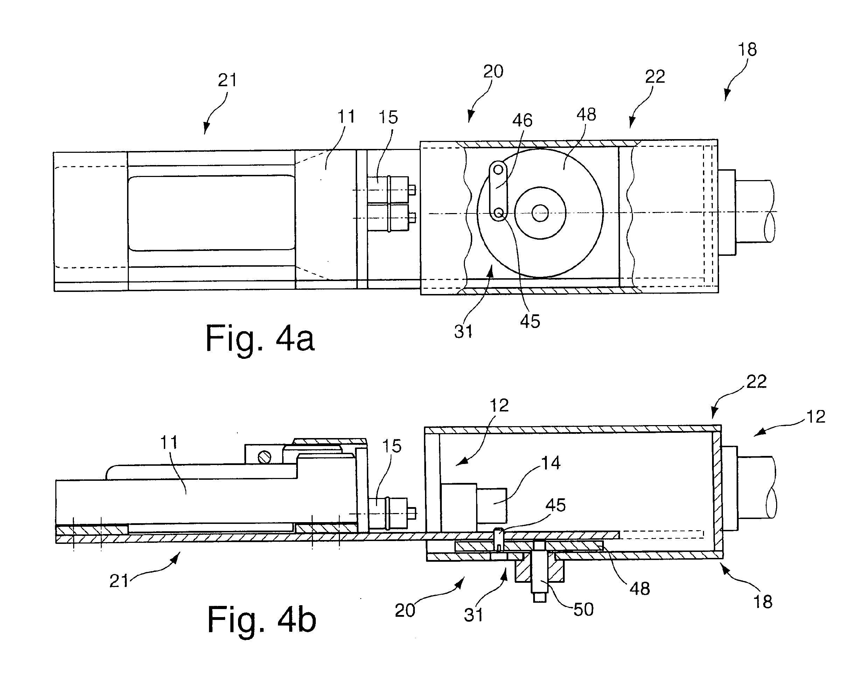

In Figur 4a und b ist eine schematische Draufsicht und Seitenansicht im Teilschnitt einer weiteren alternativen Ausführungsform der Vorrichtung 18 dargestellt. Bei dieser Ausführungsform umfasst die Antriebseinrichtung 31 eine Kreisscheibe 48, durch welche eine Verschiebebewegung ansteuerbar ist. Hierzu weist die Kreisscheibe 48 einen Führungsstift 45 auf, der in eine Kulisse 46 eingreift. Ein solcher Antriebsmechanismus 31 kann bspw. durch einen externen Schlüssel angesteuert werden, der an einer Achse 50 der Kreisscheibe 48, insbesondere an einem Vierkantanschluss angreift. Im Übrigen gelten die Ausführungen zu den vorherigen Figuren

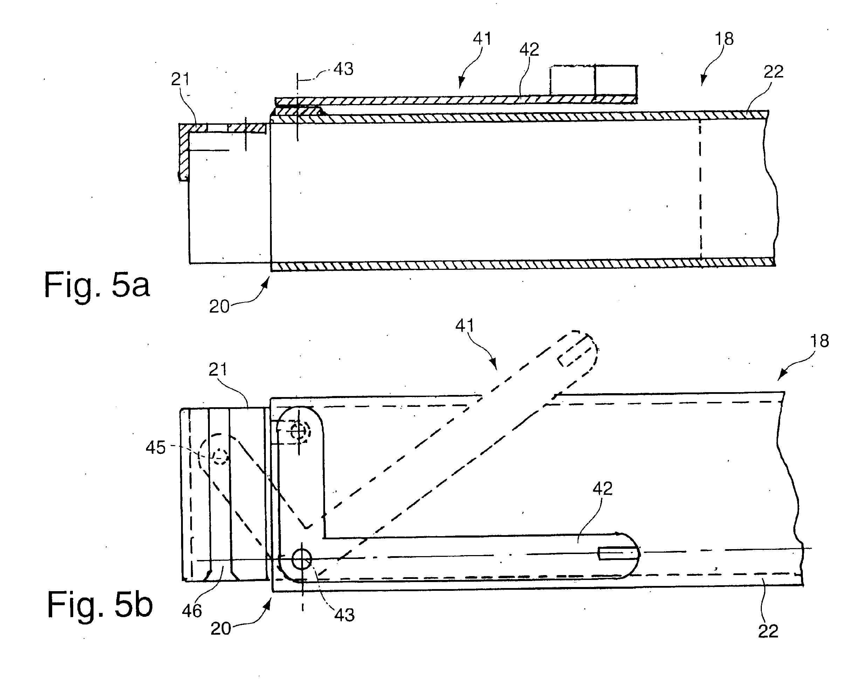

In Figur 5a ist eine alternative Ausführungsform eines Hebelmechanismus 41 zu Figur 2a dargestellt. Figur 5a zeigt eine Schnittanordnung entlang der Linie a - b in Figur 5b. Bei dieser Ausführungsform des alternativen Hebelmechanismus 41 ist der Hebelarm 42 als L-förmiger Hebel ausgebildet. Dieser kann bspw. seitlich außerhalb der Aufnahmevorrichtung 22 vorgesehen sein. Der kurze Schenkel weist wiederum einen Führungsstift 45 auf, der in eine Kulisse 46 eingreift, welche mit der Aufnahmevorrichtung 21 in Verbindung steht. Die Aufnahmevorrichtung 22 kann als U-förmiges Gehäuse ausgebildet sein, in dem die ebenfalls U-förmig ausgebildete Aufnahmevorrichtung 21 geführt ist, so dass die Schiebebewegung ausgerichtet ist. Des Weiteren kann die Aufnahmevorrichtung 22 oder 21 aus Hälften 25 gemäß Figur 1 bestehen.

In Figur 5c ist eine weitere alternative Ausführungsform einer Antriebseinrichtung 31 schematisch in einer Seitenansicht dargestellt. Bei dieser Ausführungsform ist die Antriebseinrichtung 31 als Zahnstangenantrieb 47 ausgebildet, welche eine Zahnstange 51 umfasst, die an der Aufnahmeeinrichtung 21 für die Blitzlampe 11 angebracht ist. Die Aufnahmeeinrichtung 21 kann lösbar zur Zahnstange 51 vorgesehen sein. Des Weiteren umfasst dieser Zahnstangenantrieb 47 ein Zahnrad 52, welches an der Zahnstange 51 angreift und dessen Achse in der Aufnahmevorrichtung 22 gelagert ist. Durch die Drehung des Zahnrades 52, beispielsweise über einen Drehknopf oder mittels eines externen Schlüssels, kann die Verschiebebewegung der Aufnahmevorrichtung 21 erzeugt werden. Analoges gilt für die Aufnahmevorrichtung 22. Anstelle einer manuellen Drehung des Zahnrades 52 kann auch ein motorischer Antrieb vorgesehen sein. Die Zahnstange 51 kann alternativ auch auf der Unterseite der Aufnahmevorrichtung 21 angeordnet sein. Alternativ kann auch eine Zahnstange 51 und ein Zahnrad 52 des Zahnstangenantriebs 47 auf beiden Längsseiten der Aufnahmevorrichtung 21 angeordnet sein.

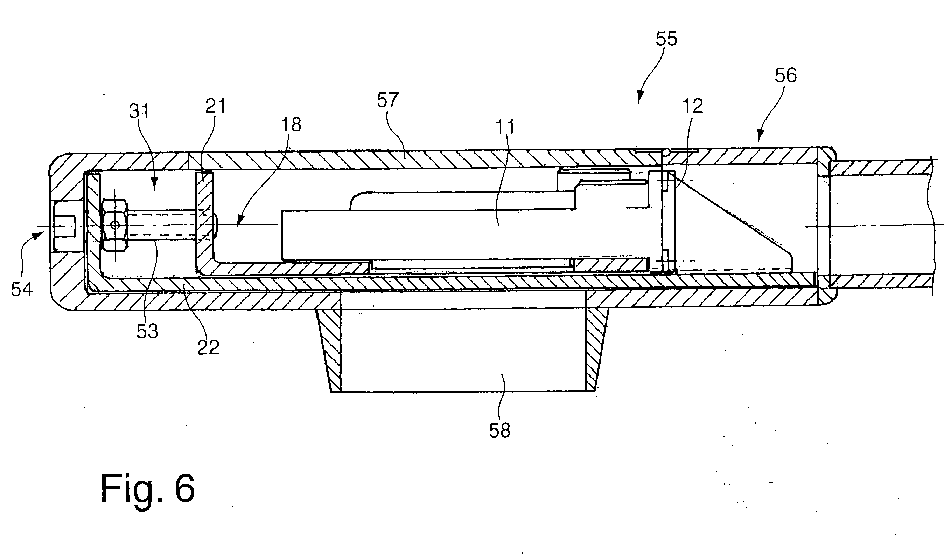

In Figur 6 ist eine schematische Schnittdarstellung einer weiteren alternativen Ausführungsform der Vorrichtung 18 dargestellt. Diese Vorrichtung 18 ist in einem Gehäuse 56 eines Handteils 55. Zur Einleitung einer Schiebebewegung zwischen der Aufnahmevorrichtung 21 und Aufnahmevorrichtung 22 ist als Antriebseinrichtung 31 ein Spindelantrieb 53 vorgesehen. Dieser Spindelantrieb 53 kann an einer Anschlussstelle 54 für ein externes Werkzeug zum Drehen des Spindelantriebes 53 aufweisen. Alternativ kann das Werkzeug auch fest daran angeordnet sein. Ein solcher Spindelantrieb 53 weist den Vorteil auf, dass besonders kleine Drehmomente zur Erzeugung einer Schiebebewegung vorgesehen sind. Die Zugänglichkeit zum Austausch der Blitzlampe 11 wird über einen Deckel 57 am Gehäuse 56 des Handgerätes 55 geschaffen, durch welchen nach dem Öffnen das Gehäuseinnere zugänglich wird. An einer Unterseite des Gehäuses 56 ist eine Austrittsöffnung 58 vorgesehen, durch welche das Pulslicht zur Behandlung der betroffenen Hautpartien treten kann.

Die vorstehend beschriebene Figuren können alternativ zur manuellen Einleitung der Schiebebewegung auch durch einen motorischen Antrieb angesteuert werden, so dass an Stelle einer erheblich kräftereduzierten Schiebebewegung durch die jeweiligen Hebelmechanismen auch eine für den Benutzer kraftfreie Schiebebewegung zum Austausch einer Blitzlampe 11 vorgesehen sein kann.

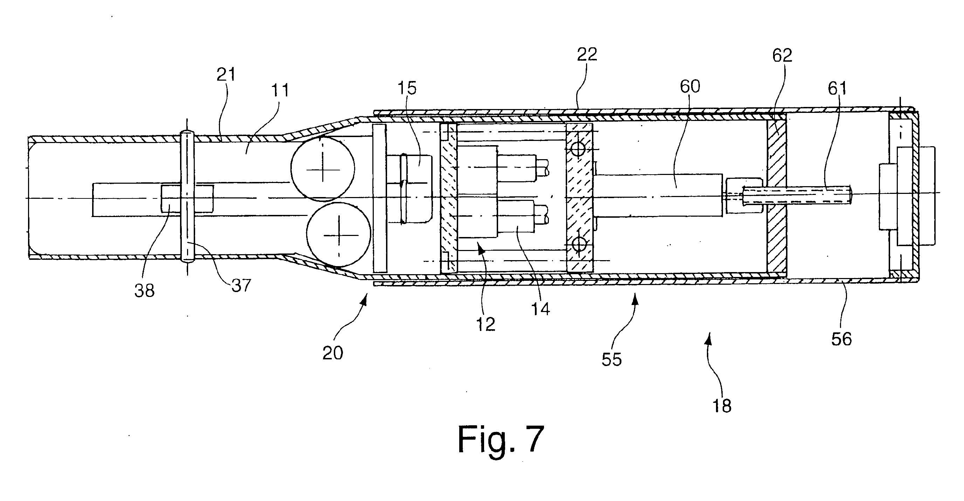

In Figur 7 ist eine schematische Draufsicht auf eine weitere alternative Vorrichtung 18 dargestellt. Diese Vorrichtung 18 weist eine Aufnahmevorrichtung 22 für die Blitzlampenaufnahme 12 auf, bei der eine Spindel 61 von einem Motor 60 angetrieben wird, um die Schiebebewegung zu erzeugen. Die Spindel 61 ist mit einer Spindelmutter 62 in Verbindung, wodurch die Aufnahmevorrichtung 21 relativ zur Aufnahmevorrichtung 22 verschiebbar ist. Die Aufnahmevorrichtung 21, 22 ist hierbei bevorzugt als Toplader ausgebildet.

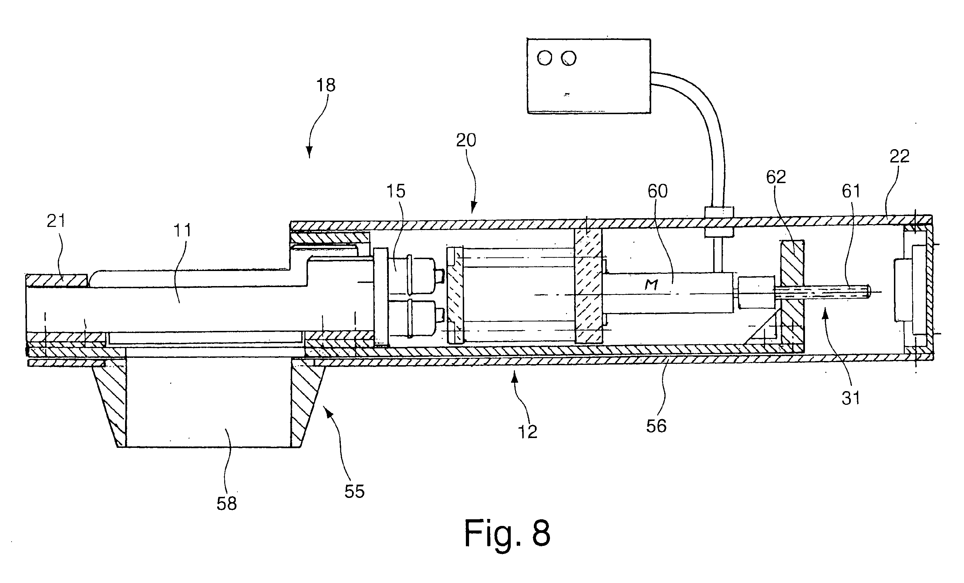

In Figur 8 ist eine schematische Seitenansicht einer alternativen Ausführungsform zu Figur 7 dargestellt. Diese Ausführungsform weicht dahingehend ab, dass die Antriebsvorrichtung 21 für die Blitzlampe 11 als Seitenlader ausgebildet ist. Im Übrigen entspricht die Ausführungsform der gemäß Figur 7. Der Motor 60 der Antriebseinrichtung 31 kann sowohl mit einem intern angeordneten Akkumulator, insbesondere einer Batterie, als auch mit einer externen Einheit zur Energieversorgung ausgestattet sein. Diese beiden Alternativen sind grundsätzlich bei allen motorisch getriebenen Antriebseinheiten 31 möglich. Darüber hinaus kann die Antriebseinrichtung 31 alternativ zum elektrischen Motor auch pneumatisch, hydraulisch oder elektromagnetisch angetrieben sein.

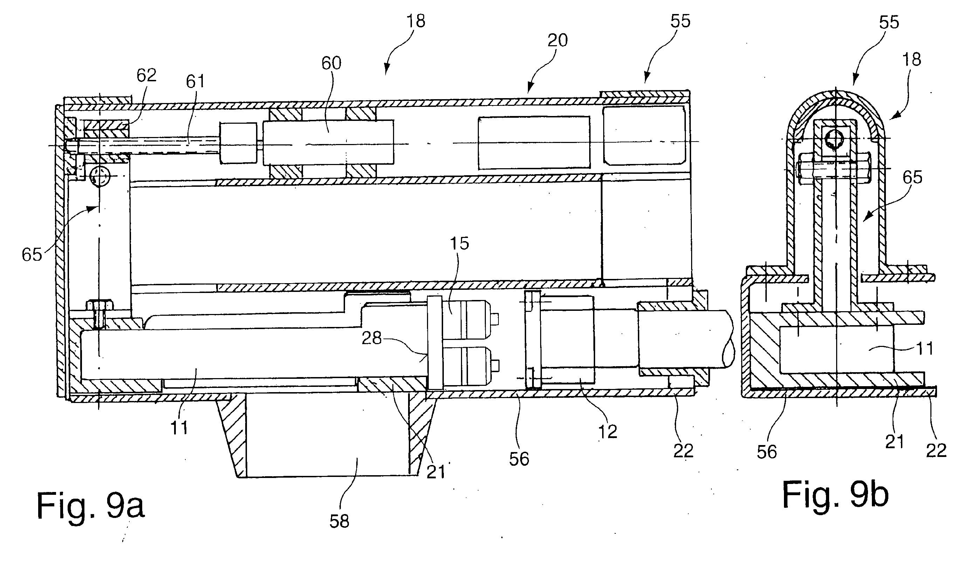

In Figur 9a ist eine schematische Schnittdarstellung einer weiteren alternativen Ausgestaltung der Vorrichtung 18 dargestellt. Figur 9b zeigt eine Schnittdarstellung entlang der Linie a - b gemäß Figur 9a. Bei dieser Ausführungsform ist die Aufnahmevorrichtung 21 der Blitzlampe 11 als Seitenlader dargestellt. Die Aufnahmevorrichtung 22 umfasst die Antriebseinrichtung 31. Beispielsweise ist über ein Gestänge 65 und einem Motor 60 eine Verschiebebewegung zwischen der Aufnahmevorrichtung 21 und der Aufnahmevorrichtung 22 vorgesehen. Sämtliche hierfür erforderlichen Komponenten können dabei dem Gehäuse 56 der Aufnahmevorrichtung 22 vorgesehen sein. Eine solche geschlossene Anordnung ermöglicht eine Integration der Vorrichtung 18 zum Wechseln einer Blitzlampe 11 in ein Handgerät 55.

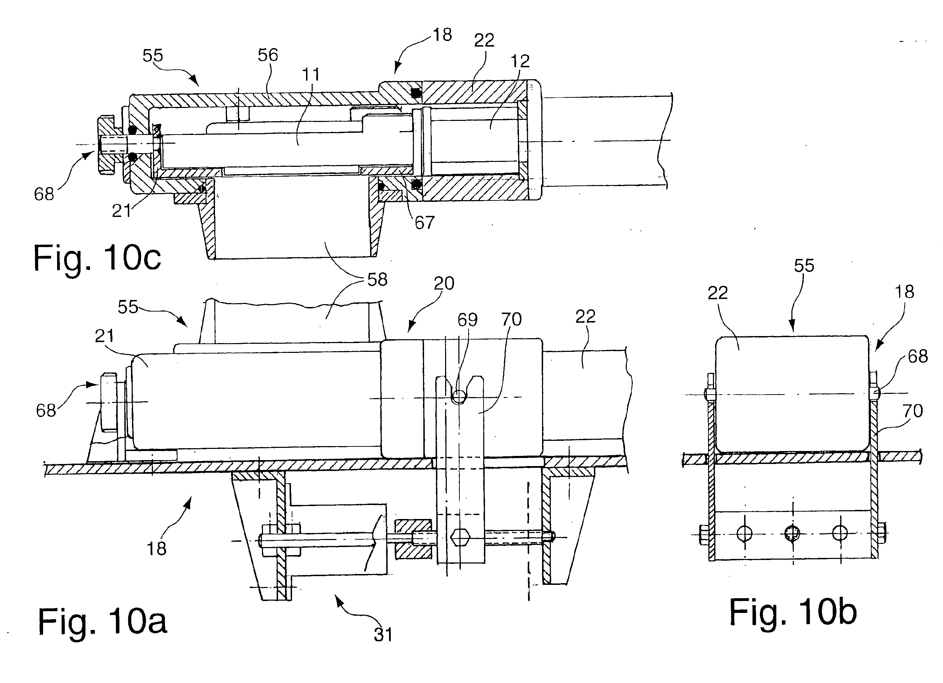

In Figur 10a und b sind schematische Seitenansichten einer weiteren alternativen Ausführungsform der Vorrichtung 18 dargestellt. Figur 10c zeigt eine schematische Schnittdarstellung der Vorrichtung 18 gemäß Figur 10a. Bei dieser Vorrichtung 18 ist ein kräftefreies Wechseln der Blitzlampe 11 ermöglicht. Die Blitzlampe 11 ist in dem Gehäuse 56 des Handgerätes 55 untergebracht. Die Vorrichtung 18 ist im Versorgungsteil für das Pulslichtgerät oder in einem externen Gerät untergebracht. Die Vorrichtung 18 trennt das Gehäuse 56 des Handgerätes 55 in zwei Hälften. Dabei stellt eine Hälfte die Aufnahmevorrichtung 22 dar. Die andere Hälfte stellt die Aufnahmevorrichtung 21 dar, wobei diese Aufnahmevorrichtung 21 bspw. zweiteilig ausgebildet ist und einen inneren Schlitten 67 aufweist, der über eine Rändelschraube 68 an der Aufnahmeeinrichtung 21 befestigt ist. Ein solches Handgerät 55 wird mittels Zapfen 69 in ein Gabelstück 70 im mittleren Bereich des Handgerätes 55 eingelegt. Durch eine Antriebseinrichtung 31 wird die Blitzlampe 11 aus der Blitzlampenaufnahme 12 gezogen. Anschließend wird nach dem Lösen der Rändelschraube 68 die Blitzlampe 11 kraftfrei aus der Aufnahmevorrichtung 21 entnommen. Das Einlegen und Einschieben der Blitzlampe 11 in die Blitzlampenaufnahme 12 geschieht in umgekehrter Reihenfolge. Beim Einlegen der Blitzlampe 11 in die Vorrichtung 18 öffnet sich eine Verriegelung der beiden Gehäusehälften.

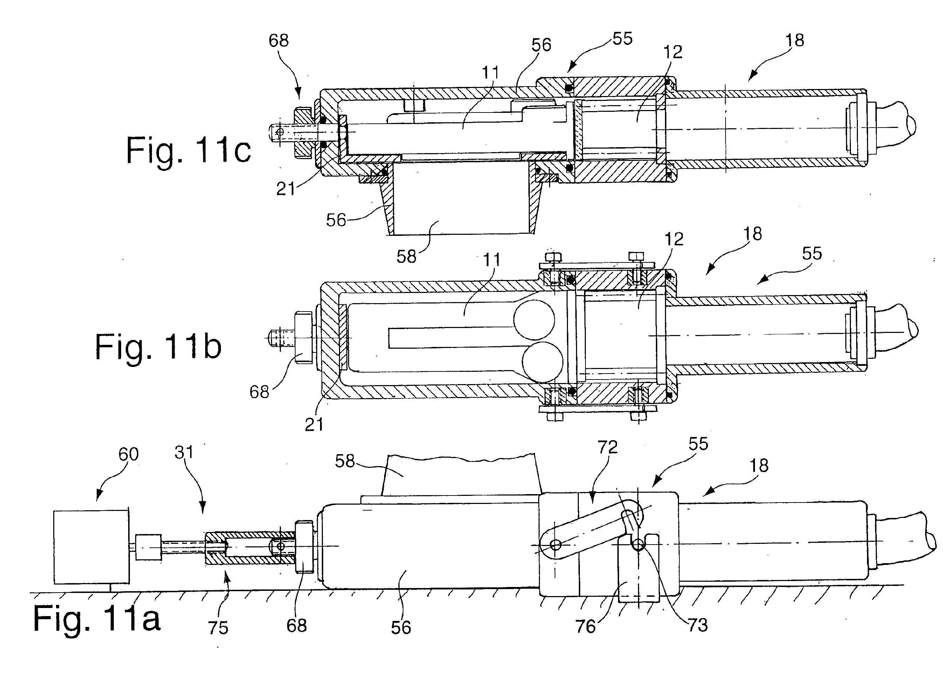

In Figur 11a ist eine schematische Seitenansicht einer weiteren alternativen Ausführungsform der Vorrichtung 18 dargestellt. Die Figuren 11b und c zeigen weitere schematische Schnittdarstellungen der Ausführungsform gemäß Figur 11a. Bei dieser Ausführungsform ist die Vorrichtung 18 wiederum in ein Handgerät 55 integriert oder Teil eines Handgerätes 55. Bei dieser Ausführungsform greift eine Verriegelungseinrichtung 72, welche an der Aufnahmevorrichtung 21 bzw. am Gehäuse 56 der Aufnahmevorrichtung 21 befestigt ist, an einem Stift 73 an, der an der Aufnahmevorrichtung 22 vorgesehen ist. Dadurch ist eine gesicherte Positionierung der Aufnahmevorrichtung 21, 22 zueinander vorgesehen. Nach dem Lösen der Verriegelungseinrichtung 72 kann die Antriebseinrichtung 31 mittels eines Motors 60 eine Schiebebewegung bewirken, so dass die Aufnahmevorrichtung 21 von der Aufnahmevorrichtung 22 abgezogen wird. Zwischen dem Motor 60 und der Aufnahmevorrichtung 21 ist eine Kupplung 75 vorgesehen, so dass die Vorrichtung 18 einerseits an den Motor 60 anschließbar ist und andererseits über den Stift 73 in einer Halterung 76 fixiert wird, welche wie der Motor 60 ortsfest vorgesehen sind.

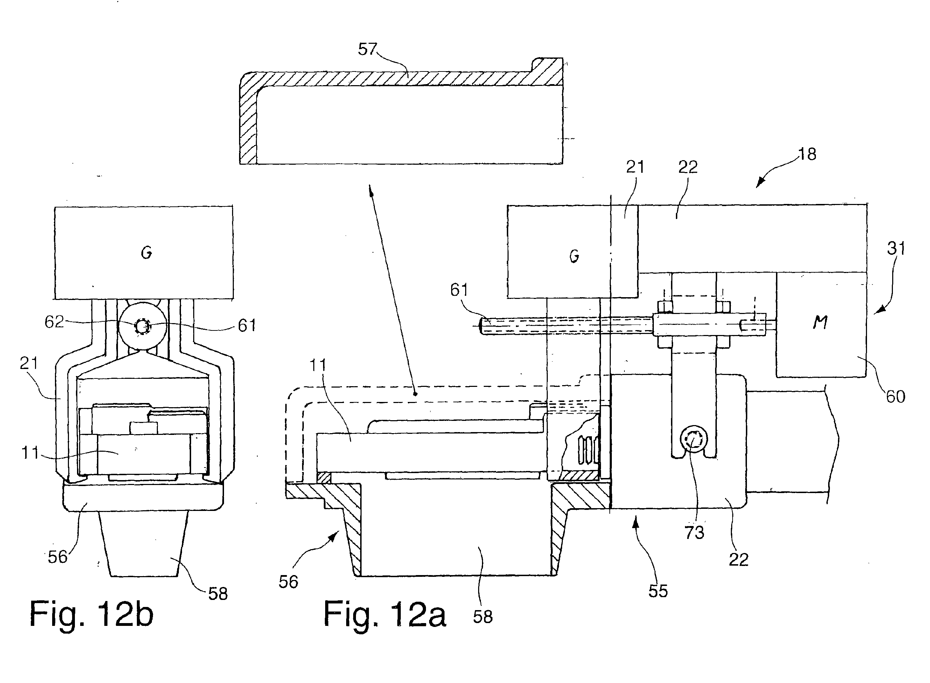

In Figur 12a und 12b ist eine weitere alternative Ausführungsform einer Vorrichtung 18 vorgesehen, welche an dem Handgerät 55 angreift, um einen Austausch der Blitzlampe 11 zu ermöglichen. Diese Vorrichtung 18 ist als separates Werkzeug ausgebildet und kann zum Handgerät 55 positioniert werden. Zum Wechseln der Blitzlampe 11 wird zunächst ein Deckel 57 des Gehäuses 56 vom Handgerät 55 geöffnet oder entfernt. Anschließend wird eine Aufnahmevorrichtung 21 an der Blitzlampe 11 positioniert. Diese Aufnahmevorrichtung 21 kann bspw. zwei Greifer umfassen, welche nach der Positionierung zur Blitzlampe 11 unter Krafteinfluss form- und kraftschlüssig daran angreifen. Anschließend wird über eine Antriebseinrichtung 31 mit einem Motor 60 eine Spindel 61 angetrieben, wodurch sich die Aufnahmevorrichtung 21 gegenüber der Aufnahmevorrichtung 22 entfernt. Somit wird über die Spindel 61 und die Spindelmutter 62, welche an der Aufnahmevorrichtung 21 vorgesehen ist, die Schiebebewegung eingeleitet. Durch die Abstützung der Vorrichtung 18 an eine Halterung oder an einem Stift 73, der am Gehäuse 56 des Handgerätes 55 vorgesehen ist, erfolgt eine definierte Positionierung der Vorrichtung 18 zum Handgerät 55 des Pulslichtgerätes. Das Montieren der Blitzlampe 11 erfolgt in umgekehrter Reihenfolge.

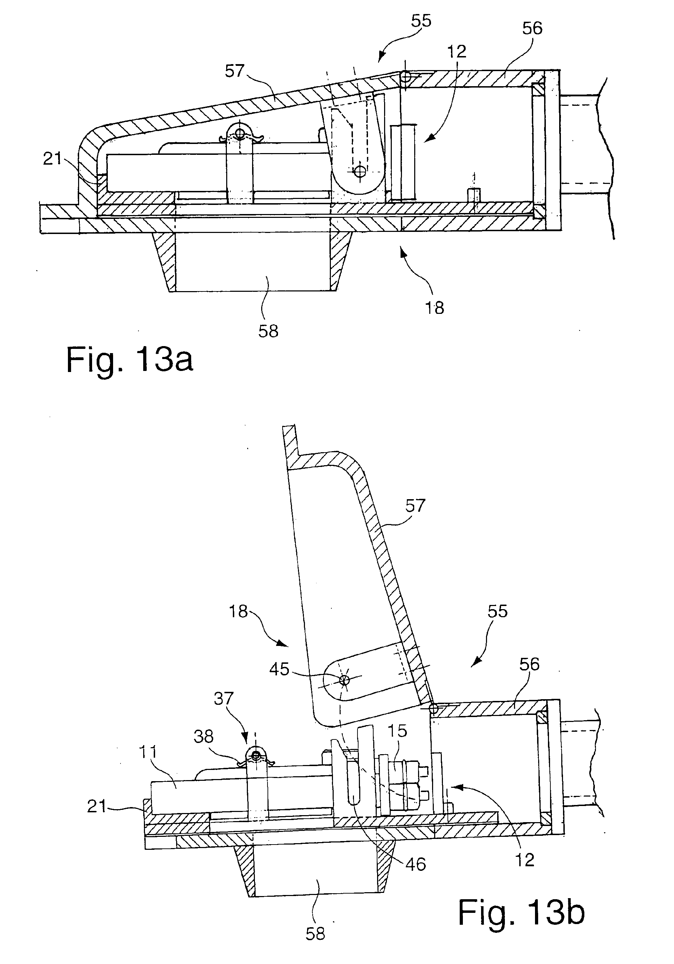

In Figur 13a und b ist eine schematische Schnittdarstellung einer alternativen Ausführungsform des Handgerätes 55 dargestellt. Dieses umfasst wiederum ein Gehäuse 56 mit einem öffenbaren Deckel 57. Bei dieser Ausführungsform erfolgt gleichzeitig mit einer Öffnungsbewegung des Deckels 57 ein Lösen und Verbinden der Blitzlampe 11 von oder zur Blitzlampenaufnahme 12. Hierfür ist an dem Deckel 57, der Teil der Aufnahmevorrichtung 22 ist, ein Führungsstift 45 vorgesehen, der an einer Kulisse 46 der Aufnahmevorrichtung 21 angreift. Durch die Schwenkbewegung wird eine Schiebebewegung erzeugt. Nach dem Lösen der Verriegelungseinrichtung 37 kann die Blitzlampe 11 nach oben entnommen werden. Diese Ausführungsform weist den Vorteil auf, dass gleichzeitig mit dem Öffnen des Deckels 57 bzw. Schließen des Deckels 57 eine Montage bzw. Demontage der Blitzlampe 11 erfolgt.

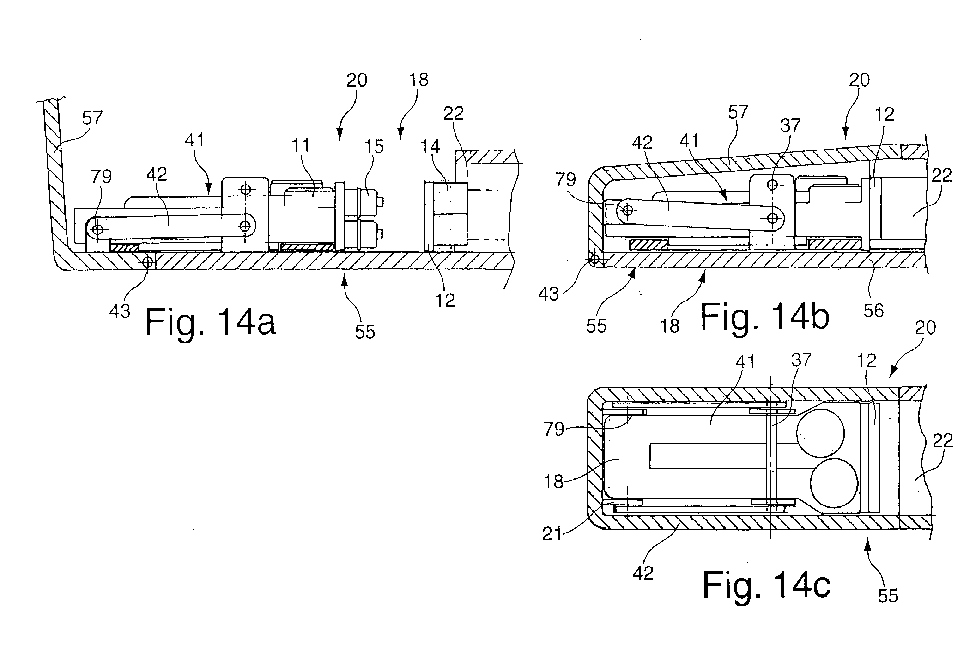

In den Figuren 14a bis c sind schematische Schnittdarstellungen einer weiteren alternativen Ausführungsform der Vorrichtung 18 dargestellt. Diese Vorrichtung 18 arbeitet in Analogie zur Vorrichtung 18 gemäß Figur 13. Bei Öffnen des Deckels 57 vom Gehäuse 56 des Handgerätes 55 erfolgt über einen Hebelmechanismus 41 ein Lösen oder Verbinden der Blitzlampe 11 zur Blitzlampenaufnahme 12. Durch die Länge des Hebelarmes 42 bzw. des Abstandes eines Gelenkpunktes 79 zur Schwenkachse 43 des Deckels 57 am Gehäuse 56 wird wiederum die Kraftreduzierung zum Lösen und Verbinden der Blitzlampe 11 an der Blitzlampenaufnahme 12 eingestellt.

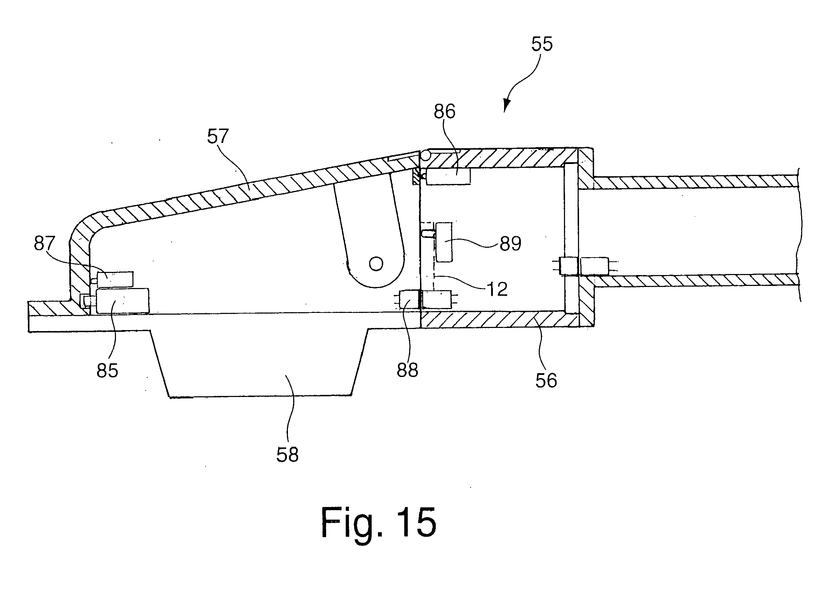

In Figur 15 ist eine weitere schematische Schnittdarstellung der Ausführungsform gemäß Figur 13 dargestellt. Bei dieser Darstellung sind lediglich Komponenten dargestellt, die für einen sicheren Betrieb des Handgerätes 55 eingesetzt werden können. Bspw. sind ein oder mehrere Hubmagnete 85 vorgesehen, die ein Öffnen des Deckels 57 vom Gehäuse 56 so lange verhindern, bis alle elektrischen Anschlüsse im Handgerät 55 spannungsfrei sind und das Kühlwassersystem drucklos ist. Zusätzlich können Schalter 86, 87 vorgesehen sein, die beim Öffnen des Deckels 57 betätigt werden. Diese können benutzt werden, um die elektrischen Anschlüsse im Handgerät 55 spannungsfrei zu schalten und um das Kühlwassersystem drucklos zu schalten. Darüber hinaus kann das Öffnen des Deckels 57 erkannt und erfasst werden. Das unbeabsichtigte Öffnen des Deckels 57 kann über weitere Sensoren, die mit beliebigen physikalischen Prinzipien arbeiten, erkannt werden und in entsprechenden Schutzmaßnahmen umgesetzt werden. Bevorzugt ist vorgesehen, dass zum Erkennen des Öffnens des Deckels 57 ein Sensor 88 als Ruhestromschleife ausgeführt ist. Gleichzeitig wird bei Betrieb des Handgerätes 55 vom Pulslichtgerät überprüft, ob die Blitzlampe 11 in der Blitzlampenaufnahme 12 vollständig steckt. Dabei kann eine Überprüfung mittels eines Schalters 89, mittels einer Ruhestromschleife, mittels eines Kraftsensors oder mittels eines auf einem beliebigen anderen physikalischen Prinzip beruhenden Schalters erfolgen.

## Patentansprüche

1. Vorrichtung zum Wechseln einer Blitzlampe (11) an einer Blitzlampenaufnahme (12), welche zumindest einen Aufnahmeanschluss (15) aufweist, der von zumindest einem Sockelanschluss (14) der Blitzlampenaufnahme (12) aufgenommen wird, wobei die Blitzlampe (11) und die Blitzlampenaufnahme (12) im montierten Zustand durch eine lösbare Steckverbindung zueinander positioniert sind, **dadurch gekennzeichnet, dass** eine Handhabungseinrichtung (20) zumindest eine Aufnahmevorrichtung (21) für eine Blitzlampe (11) und zumindest eine weitere Aufnahmevorrichtung (22) für eine Blitzlampenaufnahme (12) umfasst, die relativ zueinander durch eine Schiebebewegung verfahrbar sind und dass die Schiebebewegung von der zumindest einen Aufnahmeeinrichtung (21, 22) zum Lösen und Verbinden der Blitzlampe (11) und der Blitzlampenaufnahme (12) durch eine gemeinsame Antriebseinrichtung (31) ansteuerbar ist und dass zumindest eine der Aufnahmevorrichtungen (21, 22) zum kraftfreien Einlegen der Blitzlampe (11) oder Blitzlampenaufnahme (12) ausgebildet ist.

2. Vorrichtung nach Anspruch 1, **dadurch gekennzeichnet, dass** die Aufnahmevorrichtung (21) zumindest eine Auflagefläche (26) aufweist, an welcher zumindest eine Anlagefläche (24, 28) der Blitzlampe (11) zur zumindest formschlüssigen Aufnahme der Blitzlampe (11) in der Aufnahmevorrichtung (21) angreift.

3. Vorrichtung nach Anspruch 1, **dadurch gekennzeichnet, dass** die Aufnahmevorrichtung (22) zumindest eine Auflagefläche (26) aufweist, an welcher zumindest eine Anlagefläche (27) der Blitzlampenaufnahme (12) zur zumindest formschlüssigen Aufnahme der Blitzlampenaufnahme (12) in der Aufnahmevorrichtung (22) aufgreift.

4. Vorrichtung nach einem der vorhergehenden Ansprüche, **dadurch gekennzeichnet, dass** die Aufnahmevorrichtung (21) zumindest einen Gehäuseabschnitt zum seitlichen kraftfreien Einschieben oder zumindest einen Gehäuseabschnitt zum kraftfreien Einlegen von oben oder zumindest zwei einen Gehäuseabschnitt bildenden Hälften zum kraftfreien Positionieren zwischen den beiden Hälften aufweist.

5. Vorrichtung nach einem der vorhergehenden Ansprüche, **dadurch gekennzeichnet, dass** zwischen den zumindest zwei Aufnahmevorrichtungen (21, 22) eine Führung (32) vorgesehen ist, welche die Schiebebewegung entlang einer gemeinsamen Längsachse der zumindest zwei Aufnahmevorrichtungen (21, 22) führt.

6. Vorrichtung nach einem der vorhergehenden Ansprüche, **dadurch gekennzeichnet, dass** die Aufnahmevorrichtung (21, 22) eine Verriegelungseinrichtung (37) zur Fixierung der Blitzlampe (11) und der Blitzlampenaufnahme (12) zur jeweiligen Aufnahmevorrichtung (21, 22) aufweist.

7. Vorrichtung nach einem der vorhergehenden Ansprüche, **dadurch gekennzeichnet, dass** die Antriebseinrichtung (31) einen Hebelmechanismus (41) zur Kraftreduzierung der Schiebebewegung beim Lösen und Verbinden der lösbaren Steckverbindung zwischen der Blitzlampe (11) und der Blitzlampenaufnahme (12) umfasst.

8. Vorrichtung nach Anspruch 7, **dadurch gekennzeichnet, dass** der Hebelmechanismus (41) einen an der Aufnahmevorrichtung (21, 22) angeordneten Hebelarm (42) aufweist, dessen Schwenkachse (43) rechtwinklig zur Schiebebewegung ausgerichtet ist und eine Schiebebewegung eines Führungsstiftes (45) ansteuert, der mit der weiteren Aufnahmevorrichtung (21, 22) verbunden ist.

9. Vorrichtung nach Anspruch 7, **dadurch gekennzeichnet, dass** der Hebelmechanismus (41) durch eine Kreisscheibe (48) mit einem in eine Kulisse (46) eingreifenden Führungsstift (45) ausgebildet ist.

10. Vorrichtung nach einem der Ansprüche 1 bis 6, **dadurch gekennzeichnet, dass** die Antriebseinrichtung (31) als Spindelantrieb (53) oder Zahnstangenantrieb (47) mit einer Zahnstange (51) und einem Zahnrad (52) ausgebildet ist.

11. Vorrichtung nach einem der Ansprüche 1 bis 6, **dadurch gekennzeichnet, dass** die Antriebseinrichtung (31) einen Antriebsmotor (60) aufweist, der eine Schiebebewegung zwischen den zumindest zwei Antriebsvorrichtungen (21, 22) mit einem Spindelantrieb (53), einem Zahnstangenantrieb (47), einem Hebelmechanismus (31) oder einem Kurvengetriebe (48, 49) antreibt.

12. Vorrichtung nach Anspruch 11, **dadurch gekennzeichnet, dass** der Antriebsmotor (60) elektrisch, hydraulisch, pneumatisch oder elektromagnetisch ansteuerbar ist.

13. Vorrichtung nach einem der vorhergehenden Ansprüche, **dadurch gekennzeichnet, dass** an der die Blitzlampenaufnahme (12) aufnehmenden Aufnahmevorrichtung (22) ein Antriebsmotor (60) vorgesehen ist.

14. Vorrichtung nach einem der vorhergehenden Ansprüche, **dadurch gekennzeichnet, dass** die Handhabungseinrichtung (20) zumindest einen Sensor umfasst, der nach dem vollständigen Verbinden der Blitzlampe (11) und der Blitzlampenaufnahme (12) und dem Schließen des Deckels (57) des Gehäuses (56) den Deckel (57) verriegelt, und die Versorgung der Blitzlampe (11) mit Kühlfluid und elektrischem Strom frei gibt.

15. Vorrichtung nach einem der vorhergehenden Ansprüche, **dadurch gekennzeichnet, dass** die Handhabungseinrichtung (20) zumindest einen Sensor aufweist, der die Schiebebewegung der zumindest einen Aufnahmevorrichtung (21, 22) oder ein Öffnen des Deckels (57) des Gehäuses (56) frei gibt, sobald die Blitzlampe (11) spannungsfrei und deren Kühlwasserschläuche drucklos geschaltet sind.
